# EUROPÄISCHE PATENTANMELDUNG

(11) **EP 0 930 084 A1**
(43) Veröffentlichungstag der Anmeldung: **21.07.1999**
(21) Anmeldenummer: 98100812.1
(22) Anmeldetag: 19.01.1998
(51) Int. Cl.: A61N 1/36

(54) **Elektromedizinische Vorrichtung zur funktionellen Stimulation**

(71) Anmelder: Leubner, Manfred, Dr., 94560 Offenberg (DE); Fischermeier, Martin, 94539 Grafling (DE); Kreter, Michael, 94474 Vilshofen (DE); Schosser, Johann, 94530 Auerbach (DE); Zelenka, Alois, 94136 Thyrnau (DE)
(72) Erfinder: Leubner, Manfred, Dr., 94560 Offenberg (DE); Fischermeier, Martin, 94539 Grafling (DE); Kreter, Michael, 94474 Vilshofen (DE); Schosser, Johann, 94530 Auerbach (DE); Zelenka, Alois, 94136 Thyrnau (DE)
(74) Vertreter: Muschke, Markus, Dipl.-Phys.

(57) **Zusammenfassung**

Die Erfindung betrifft eine elektromedizinische Vorrichtung zur funktionellen Stimulation von Muskel- und/oder Nervengewebe. Die Vorrichtung umfaßt einen Oszillator, der ein niederfrequentes Stromimpuls-Frequenzmuster erzeugt, das über zumindest eine Kontaktelektrode an das funktionell zu stimulierende Muskel- und/oder Nervengewebe angelegt wird. Bevorzugt wird das Gerät netzunabhängig betrieben. Das Gerät kann auch zur intentionsabhängigen Stimulierung eingesetzt werden, wozu es über einen Triggereingang verfügt, an den ein von einer Muskel- und/oder Nervenpartie abgegriffenes Spannungssignal angelegt wird. Bevorzugt wird das Gerät zur Stimulierung bzw. zum Training des Musculus corpus cavernosum bzw. des Musculus temporalis verwendet.

## Beschreibung

Die vorliegende Erfindung betrifft eine elektromedizinische Vorrichtung zur funktionellen Stimulation von Muskel- und/oder Nervengewebe gemäß Patentanspruch 1, die Verwendung einer solchen Vorrichtung gemäß den Patentansprüchen 7 bis 9 sowie ein Verfahren zur Verbesserung der körperlichen Erscheinung einer erwachsenen, männlichen Person gemäß Patentanspruch 10.

Elektromedizinische Geräte zur Reizstrombehandlung werden üblicherweise zur Stimulation bzw. zum Aufbau von Muskelgruppen eingesetzt, beispielsweise in der Rehabilitation oder bei älteren Menschen, denen eine gezielte Aktivierung gewisser Muskel- oder Nervengruppen mit zunehmendem Alter zusehends schwerer fällt.

Zur Reizstrombehandlung werden auf die Haut einer Person Kontaktelektroden aufgebracht, an die üblicherweise Stromimpulse mit Frequenzen von deutlich mehr als 50 Hz angelegt werden. Stromimpulse mit geeignet gewählter Amplitude bewirken eine Kontraktion von Muskelgruppen in der Nähe der Kontaktelektroden. Hierzu wird üblicherweise ein konstantes Frequenzmuster für die Stromimpulse verwendet. Herkömmliche Geräte zur Reizstrombehandlung sind relativ groß und sperrig.

Die Erfindung betrifft ferner die Verwendung einer elektromedizinischen Vorrichtung zur Verbesserung der körperlichen Erscheinung bzw. des Wohlbefindens einer erwachsenen, männlichen Person. Erwachsene, männliche Personen leiden zum Teil an einer erektilen Dysfunktion (mangelnde Versteifung des Gliedes), was zumeist Impotenz hervorruft (Unfähigkeit, den Geschlechtsverkehr zu vollziehen). Eine erektile Dysfunktion wird häufig durch eine Störung bzw. Schwäche des Muskulus corpus cavernosum hervorgerufen. Dieser Muskel leitet die Erektion (Versteifung) ein und unterhält sie, da er selektiv für die Füllung des Schwellkörpers sorgt, was für die Erektion unerläßlich ist.

Zur Behebung der erektilen Dysfunktion werden zumeist therapeutische und/oder chirurgische Maßnahmen vorgeschlagen. Beispielsweise ist das gezielte Spritzen von Medikamenten in das Penisgewebe hinein bekannt, was jedoch mit Schmerzen und unerwünschten Nebenwirkungen, bis hin zur Dauererektion, und Unverträglichkeiten verbunden ist, die durch die bescheidene Erfolgsrate nicht gerechtfertigt erscheinen. Auch das chirurgische Einsetzen von Implantaten in das Penisgewebe ist bekannt; diese führen jedoch häufig zu einer unerwünschten Narbenbildung und können nur schwer und unter erheblicher Narbenbildung wieder aus dem Penis entfernt werden.

Allen diesen Verfahren ist gemeinsam, daß es sich um therapeutische Maßnahmen handelt. Zur Behandlung ist hierzu vorab ein vertrauliches Gespräch mit einem Arzt oder Therapeuten über ein sehr intimes Thema unabdingbar, was bei den betroffenen Personen zumeist dazu führt, daß sie einem solchen Gespräch und somit auch einer Behandlung aus dem Wege gehen.

Es ist deshalb Aufgabe der vorliegenden Erfindung, Geräte bzw. die Verwendung solcher Geräte zur funktionellen Stimulation von Muskel- und/oder Nervengewebe zu verbessern. Außerdem soll ein Verfahren zur Verbesserung der körperlichen Erscheinung insbesondere von erwachsenen, männlichen Personen geschaffen werden. Insbesondere soll für dieses Verfahren keine therapeutische Behandlung notwendig sein.

Diese Aufgabe wird gelöst durch eine elektromedizinische Vorrichtung gemäß Patentanspruch 1, durch die Verwendung einer solchen Vorrichtung gemäß den Patentansprüchen 7 bis 9 sowie durch ein Verfahren gemäß dem Patentanspruch 10.

Hierzu umfaßt ein elektromedizinisches Gerät einen Oszillator, der Stromimpulse erzeugt. Das Ausgangssignal wird, ggf. nach weiterer Verstärkung im Gerät, an Kontaktelektroden weitergeleitet, die auf einer Körperstelle einer Person angebracht werden, um für eine funktionelle Stimulation von Muskel- und/oder Nervengewebe zu sorgen. Vorzugsweise wird eine Muskel- und/oder Nervenpartie nur gezielt bzw. lokal und mit gewünschter Wirkung auf den Organismus stimuliert bzw. trainiert.

Dabei weist das Gerät ferner insbesondere eine eigene, netzunabhängige Spannungsversorgung wie z.B. eine Batterie oder einen Akku auf. Hierdurch wird vorteilhaft ein netzunabhängiger Betrieb des Gerätes möglich, beispielsweise auf der Arbeit, zu Hause oder unterwegs.

Vorzugsweise gibt das elektromedizinische Gerät die Stromimpulse in Form niederfrequenter Frequenzmuster ab, vorzugsweise im Bereich unterhalb von 100 Hz, wodurch der erfindungsgemäße, überraschend wirkungsvolle Stimulierungs- bzw. Trainingseffekt bewirkt wird. Vorzugsweise ist das sogenannte Austastverhältnis, d.h. das Verhältnis der Dauer eines Einzelimpulses zum zeitlichen Abstand benachbarter Impulse, klein, zweckmäßig kleiner als oder etwa gleich 0,25 und vorzugsweise kleiner als 0,01. Auch ein Austastverhältnis von bis zu 10⁻⁴ hat sich als sehr wirkungsvoll zum gezielten Aufbau von Muskelgruppen erwiesen.

Als besonders trainingswirksam hat sich ein relativ langsames Ansteigen bzw. Abfallen der Stromimpulse, vorzugsweise in Trapezform, erwiesen. Bevorzugt wird dabei eine Anstiegszeit von etwa 25% der Gesamtimpulsdauer angesehen.

Vorzugsweise gibt das elektromedizinische Gerät ein vorbestimmtes Frequenzmuster aus, d.h. Stromimpulse mit vorbestimmter Impulsform, Impulsdauer, Anstiegszeit und Impulsfrequenz. Je nach den Erfordernissen des Benutzers bzw. der Anwendung kann das Frequenzmuster angepaßt werden, beispielsweise durch Änderung der Amplitude oder der Frequenz der Stromimpulse oder durch Einstellen eines Intervallbetriebs der Stromimpulse.

Es hat sich gezeigt, daß ein Frequenzmuster je nach Anwendung bzw. Trainingszweck unterschiedlich wirksam ist. Deshalb sieht eine bevorzugte Ausführungsform eines erfindungsgemäßen Geräts vor, daß ein vorbestimmtes Frequenzmuster je nach Anwendung auf ein anderes vorbestimmtes Frequenzmuster geändert werden kann. Auf diese Weise wird vorteilhaft ein multifunktionelles Gerät realisiert, das je nach Geräteeinstellung zur Stimulation unterschiedlicher Muskel- und/oder Nervengruppen dienen kann.

Vorteilhaft ist, daß der Benutzer frei in der Verwendung des Gerätes ist, weil das Gerät Mindeststandards, insbesondere gesetzliche Sicherheitsauflagen für elektromedizinische Geräte, einhält. Vorteilhaft ist ferner, daß zum Training bzw. zur Stimulation keine Behandlung oder Therapie durch einen Arzt oder Therapeuten nötig ist; vielmehr kann ein Benutzer den Trainingsumfang oder die Stimulationsintensität den eigenen Bedürfnissen anpassen. Dies erspart dem Benutzer peinliche Gespräche über intime Probleme mit einem Arzt oder Therapeuten, was darüber hinaus auch zu einer beträchtlichen Kostenersparnis führt. Auch kann das Training vorteilhaft dem individuellen Tagesablauf angepaßt werden.

Ferner sieht eine weitere erfindungsgemäße Ausführungsform einen Geräteeingang oder Triggereingang und zumindest eine Meßelektrode vor. Diese Meßelektrode dient dem Abgriff eines Nervenimpulses bzw. Spannungssignals von einer Körperstelle des Benutzers mit einer darunter befindlichen Muskel- und/oder Nervenpartie. Dieser Nervenimpuls wird vom Benutzer vorzugsweise intentionsabhängig zur Aktivierung oder Ausführung einer körperlichen Funktion erzeugt. Über eine Triggerfunktion des Gerätes wird vorteilhaft eine intentionsabhängige Steuerung des Gerätes bewirkt. Der abgegriffene Nervenimpuls kann von der Person jedoch auch unbewußt erzeugt werden, und mit Hilfe der Triggerfunktion des Gerätes einen körperlichen Effekt auslösen. Der dann seinerseits vom Gerät erzeugte Stromimpuls bzw. die Stromimpulsfolge dient der Aktivierung oder Ausführung der körperlichen Funktion.

Vorzugsweise wird zum Triggerbetrieb der abgegriffene Spannungs- bzw. Nervenimpuls im Gerät verstärkt, ggf. gefiltert, und dann als Triggersignal für den Oszillator des Gerätes verwendet. Überschreitet das abgegriffene und ggf. verstärkte Spannungssignal einen einstellbaren Schwellenwert, so wird vom Gerät das durch den Benutzer gewählte Frequenzmuster abgegeben, welches über die Kontaktelektroden zur Stimulation von Muskel- und/oder Nervengewebe führt.

Vorzugsweise sind Batterieeingang und Ausgang des Oszillators bzw. des Gerätes galvanisch getrennt. Auch ist der Ausgang vorzugsweise verriegelungssicher, so daß bei Anschluß der Kontaktelektroden an das Gerät nicht sofort die gewählte Maximalamplitude an der Kontaktelektrode anliegt.

Bevorzugt wird das erfindungsgemäße Gerät zur gezielten, lokalen Stimulation bzw. zum Aufbau des Muskulus corpus cavernosum bei einer erwachsenen, männlichen Person verwendet. Hierzu wird zumindest eine Kontaktelektrode in der Nähe der Peniswurzel angebracht. Ein geeignet auf diesen Muskel abgestimmtes Frequenzmuster wird vom Oszillator erzeugt und an die Kontaktelektrode abgegeben. Dadurch kommt es zu einer funktionellen Stimulation des Muskulus Corpus Cavernosum (Mcc), der vor und während des Geschlechtsverkehrs eine Füllung des Schwellkörpers bewirkt.

Bei einer möglichen Verwendung des Gerätes erfolgt die Stimulation unmittelbar vor und/oder während des Geschlechtsverkehrs, wobei die kosmetische Wirkung der Verwendung bzw. des Verfahrens zum Tragen kommt, nämlich aufgrund der verbesserten, weil männlicheren körperlichen Erscheinung der Person.

Bevorzugt wird der Mcc jedoch nicht zum Geschlechtsverkehr sondern mehrmals täglich bzw. je nach den Bedürfnissen des Benutzers stimuliert, was zu einem gezielten Aufbau bzw. einer gezielten Stärkung dieses Muskels führt. Anders als bei therapeutischen Verfahren bedarf die Person für dieses Training keiner Konsultation durch einen Arzt oder Therapeuten, so daß keine intimen Probleme besprochen werden müssen. Die erfindungsgemäße Wirksamkeit von Stromimpulsen zur Stärkung des Mcc war bisher nicht bekannt und so auch nicht erwartet worden.

Vorteilhaft ist ferner, daß es zum Geschlechtsverkehr keiner umständlichen Manipulation und keines Anlegens von Hilfsmittel jeglicher Art bedarf, da ja der so gestärkte Mcc automatisch zur erforderlichen Füllung des Schwellkörpers führt.

Vorteilhaft ist, daß aufgrund der netzunabhängigen Einsatzmöglichkeit des Gerätes mit Hilfe einer eigenen Spannungsversorgung diese Stimulation bei Bedarf vorgenommen werden kann, beispielsweise zu Hause, auf der Arbeit oder unterwegs, und nicht nur unmittelbar vor oder gar während des Geschlechtsverkehrs vorgenommen werden muß.

Die dadurch bewirkte verbesserte Erektionsfähigkeit führt bei erwachsenen, männlichen Personen im Bedarfsfall zu einer verbesserten körperlichen Erscheinung.

Weitere vorteilhafte Anwendungsformen des erfindungsgemäßen Gerätes, insbesondere in netzunabhängiger Betriebsweise, betreffen die Verbesserung der körperlichen Erscheinung einer Person durch gezielten Muskelaufbau durch Muskelstimulation z.B. nach einem Schlaganfall, durch gezielte Stimulation und Stärkung der Muskulatur bzw. des jeweiligen Schließmuskel der Blase oder des Mastdarms, damit es erst gar nicht zu einer Blasen- oder Mastdarminkontinenz kommt, und durch gezielte Stimulation des Musculus temporalis.

Bei einer bevorzugten Verwendung des erfindungsgemäßen Gerätes wird statt der streifenförmigen Kontaktelektroden ein zylinder- bzw. ellipsenförmiger Körper mit aufgesetzten, vorzugsweise zwei Streifenelektroden in Umfangsrichtung eingesetzt. Die beiden Streifenelektroden befinden sich hierzu erfindungsgemäß nicht am vorderen und hinteren Endbereich dieses Körpers, sondern in einem Mittelbereich, wobei der Abstand der beiden Elektroden geeignet an die zu stimulierende Muskel- bzw. Nervenpartie angepaßt ist und bevorzugt etwa 1 bis 2 cm entspricht.

Zur individuellen Anpassung des Elektroden-Körpers kann dieser in seinem Umfang variierbar sein, beispielsweise durch Aufpumpen, wobei die Elektroden dann vorzugsweise einen Umfangseinschnitt aufweisen und dehnbar ausgebildet sind. Damit der Körper nach Einführen nicht in der Körperöffnung verschwindet, ist der erfindungsgemäße Elektroden-Körper vorzugsweise an einem Ende mit einem Anschlag versehen, der bevorzugt als Gummiring ausgebildet ist, der in Axialrichtung geeignet verschoben werden kann, jedoch einer solchen Verschiebung einen geeigneten Widerstand entgegensetzt, so daß er der Anschlagsfunktion dienen kann. Vorteilhaft kann so eine individuelle Anpassung des Elektrodenkörpers vorgenommen werden.

Nachfolgend wird ein bevorzugtes Ausführungsbeispiel einer erfindungsgemäßen elektromedizinischen Vorrichtung und eines Verfahrens unter Bezugnahme auf die beigefügten Figuren beschrieben. In den Figuren zeigen:
Fig. 1 eine perspektivische Schemadarstellung eines erfindungsgemäßen elektromedizinischen Gerätes;
Fig. 2 ein Blockschaltbild eines erfindungsgemäßen elektromedizinischen Gerätes, das die wesentlichen funktionsbeteiligten Elemente zusammenfaßt;
Fig. 3 ein Funktionsschema zur Festlegung des Frequenzmusters eines erfindungsgemäßen elektromedizinischen Gerätes;
Fig. 4 ein weiteres Funktionsschema zur Festlegung des Frequenzmusters eines erfindungsgemäßen elektromedizinischen Gerätes darstellt; und
Fig. 5 zwei bevorzugte Ausführungsformen von Elektrodenkörpern darstellt, die jeweils mit zwei Elektroden in Umfangsrichtung versehen sind.

Es sei darauf hingewiesen, daß diese Figuren und die entsprechende Beschreibung, wie nachfolgend dargelegt, nur in beispielhafter und nicht etwa nur in einer auf diese eine konkrete Ausführungsform beschränkten Art und Weise auszulegen sind.

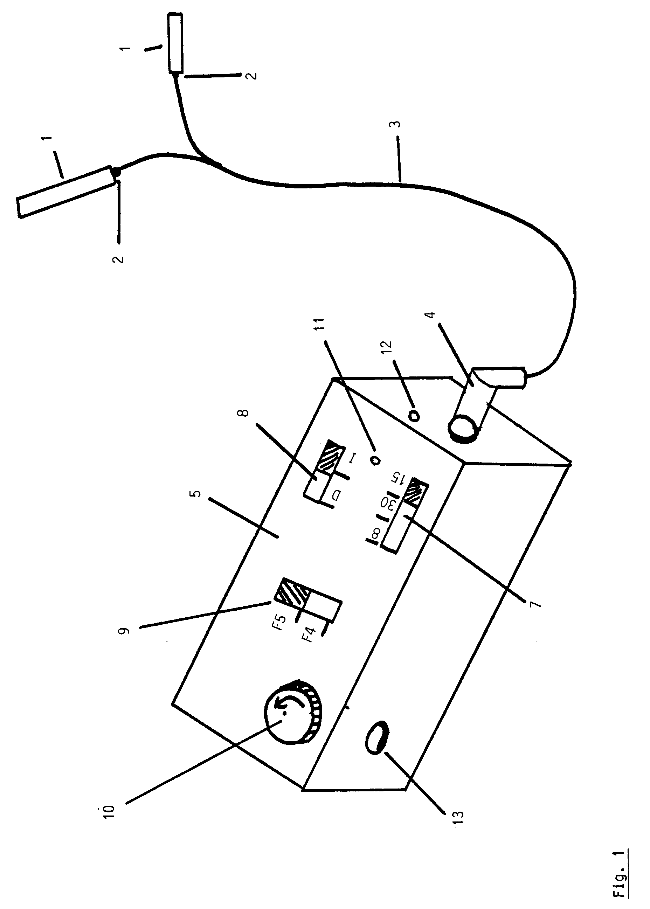

Fig. 1 zeigt eine perspektivische Schemadarstellung eines erfindungsgemäßen elektromedizinischen Gerätes.

In einem Gehäuse 5 sind ein Oszillator (nicht dargestellt) und eine netzunabhängige Spannungsversorgung (nicht dargestellt) wie etwa eine Batterie oder ein Akku mit vorzugsweise 9 Volt Betriebsspannung untergebracht. Hierdurch ist ein netzunabhängiger Betrieb möglich.

Das Gerät verfügt über einen Ausgang, vorzugsweise eine Klinkenbuchse, an dem das Ausgangssignal des Oszillators anliegt. Der Oszillator dient der Erzeugung von vorzugsweise rechteckförmigen Stromimpulsen, wie nachfolgend noch beschrieben wird, und ist bevorzugt auf einer elektronischen Schaltkarte gemeinsam mit einer Ansteuerelektronik angeordnet.

Über eine Klinkenbuchse 4 und ein vorzugsweise abgeschirmtes Verbindungskabel 3 sind die Kontaktelektroden 1 mit dem Gerät verbunden. Ein Paar von Kontaktelektroden wird auf einen Hautbereich in der Nähe eines bestimmten Muskel- und/oder Nervengewebes angebracht. Bei anderen Verwendungszwecken kann jedoch auch eine andere Zahl von Kontaktelektroden verwendet werden. Bevorzugt werden die Kontaktelektroden symmetrisch um das zu stimulierende Muskel- und/oder Nervengewebe angeordnet.

Die Kontaktelektroden sind vorzugsweise über lösbare Kontakte 2 wie etwa eine Knopf- oder Steckverbindung mit dem Verbindungskabel 3 verbunden, so daß die Kontaktelektroden zur Einhaltung von hygienischen Mindeststandards, beispielsweise bei einer aufeinanderfolgenden Verwendung bei verschiedenen Personen, ausgetauscht oder ersetzt werden können.

Leuchtdioden zeigen jeweils an, daß das Gerät eingeschaltet ist (grüne LED 11) und gerade Stromimpulse ausgibt (gelbe LED 12). Bevorzugt ist der Geräteausgang verpolungssicher (z.B. über eine Dioden- oder Transistorenschaltung) und verfügt über eine Ausgangsverriegelung gemäß DIN VDE 0750, Teil 219, Unterpunkt 51.102.

Bevorzugt ist das Gerät ausgelegt, um bei der vorgesehen Betriebsspannung von z.B. 9 Volt bei einem Hautwiderstand von 5 kOhm eine maximale Ausgangsstromstärke von 40 mA auszugeben. Unterhalb von dieser maximalen Ausgangsstromstärke kann die Amplitude der Stromimpulse beliebig eingestellt werden, wodurch die Intensität der Stimulation variiert werden kann. Ferner ist das Gerät ausgelegt, daß gemäß DIN VDE 9750, Teil 219, Unterpunkt 51.104 der Stromgrenzwert von 60 mA und eine Ausgangsspannung von 500 V bei einem Lastwiderstand von 500 Ohm nicht überschritten werden.

Zur Beeinflussung des Betriebszustands verfügt das Gerät über ein Drehpotentiometer 10, vorzugsweise mit Rastfünktion, welches in einer Betriebsart (Dauerbetrieb, Intervallbetrieb) zur Regelung der Amplitude der Stromimpulse und in einer weiteren Betriebsart (Triggerbetrieb) zur Regelung einer geräteinternen Verstärkung, wie noch beschrieben wird, dient.

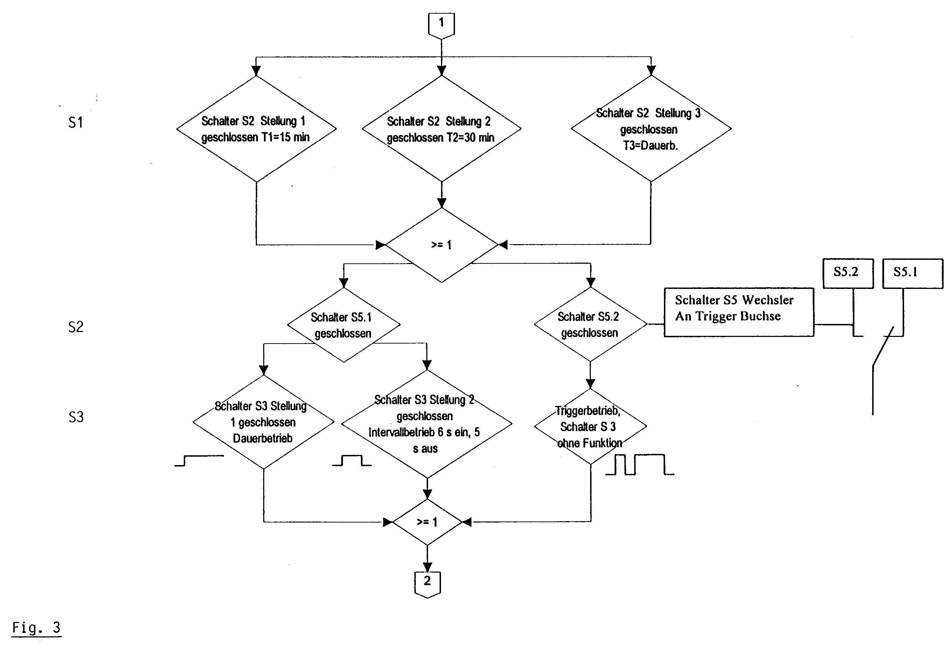

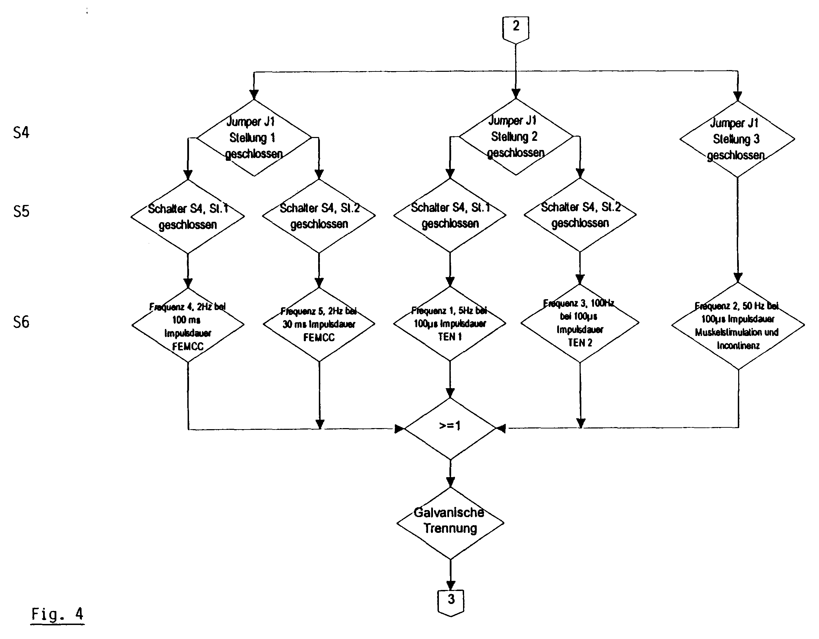

Ferner sind auf der Gehäuseoberseite 5 drei Rastschalter zur Wahl vorbestimmter Betriebszustände vorgesehen. Ein erster Schalter (Schalter 2; 7) dient der Wahl einer Betriebsdauer, ein zweiter Schalter (Schalter 3; 8) der Wahl des Betriebszustands und ein dritter Schalter (Schalter 4; 9) der Wahl des Frequenzmusters. Bei einer bevorzugten Ausführungsform, wie nachfolgend im Zusammenhang mit den Figuren 3 und 4 beschrieben, kann der Benutzer wahlweise mit Schalter 2 eine Behandlungsdauer von 15 Minuten, 30 Minuten oder eine unendliche Behandlungsdauer vorgeben und mit Schalter 3 zwischen einem Dauerbetrieb (D) und einem Intervallbetrieb (I) wählen.

Ferner kann das Gerät einen Triggereingang 13 aufweisen, an dem beim Triggerbetrieb ein Spannungssignal anliegt. Vorzugsweise sind Batterie und Geräteeingang galvanisch getrennt. Das Spannungssignal wird in üblicher Weise mit Hilfe einer nicht dargestellten Meß- bzw. Kontaktelektrode lokal von einem Muskel- und/oder Nervengewebe abgegriffen. Das abgegriffene Spannungssignal ist dabei entweder intentionsabhängig gesteuert, wobei eine gewisse Körperfunktion bzw. Muskelkontraktion bewußt bewirkt wird, wie beispielsweise die Aktivierung eines Muskels, kann jedoch auch vom Unterbewußtsein gesteuert sein, wie beispielsweise bei der Kontraktion des Muskulus corpus cavernosum (Mcc) zur Einleitung der Erektion des männlichen Gliedes oder bei der isometrischen Kontraktion der Blasenmuskulatur, insbesondere des Blasenschließmuskels, oder des Mastdarmschließmuskels.

Vorzugsweise wird das abgegriffene Spannungssignal geräteintern verstärkt und dient als Triggersignal für den Oszillator. Insbesondere zur Vermeidung von Rückkopplungseffekten wird geräteintern ein Schwellenwert vorgegeben, so daß das nachfolgend beschriebene Frequenzmuster nur dann über die Kontaktelektroden 1 an das gleiche oder an ein anderes Muskel- und/oder Nervengewebe ausgegeben wird, wenn das verstärkte Spannungssignal, ggf. nach Filterung zur Vermeidung eines Rauschens, diesen Schwellenwert übersteigt.

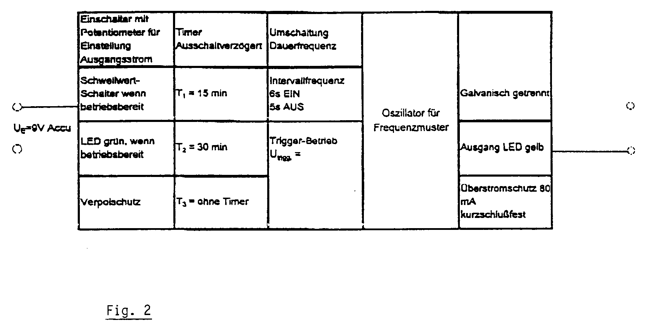

Fig. 2 faßt die wesentlichen, funktionsbeteiligten Elementgruppen und Funktionen eines erfindungsgemäßen Gerätes übersichtlich zusammen.

Zur Stimulation haben sich Frequenzmuster als besonders wirksam herausgestellt, die aus einer Folge von rechteck- bzw. trapezförmigen Stromimpulsen bestehen. Dabei beträgt die Anstiegszeit der Rechteckimpulse bevorzugt etwa 25% der Gesamtimpulsdauer. Bevorzugt wird eine im Vergleich zur Anstiegszeit sehr Kleine Abfallzeit. Bei einer weiteren Ausführungsform kann jedoch auch eine in etwa symmetrische Impulsform eingesetzt werden. Bevorzugt wird ein relativ Kleines Austastverhältnis (d.h. Verhältnis der Impulsdauer zum Abstand aufeinanderfolgender Impulse) der Stromimpulse unterhalb von 0.25 verwendet. Bevorzugt können die folgenden Betriebszustände in Form von vorbestimmten Frequenzmustern gewählt werden:
1) Rechteckimpulse mit einer Frequenz von 5 Hz und einer Impulsdauer von 100 Mikrosekunden, vorzugsweise zur transcutanen Elektronervenstimulation, insbesondere zur Stimulation des Musculus temporalis oder bei Schmerzen wie Kopfschmerzen, Nervenschmerzen oder Migräne;
2) Rechteckimpulse mit einer Frequenz von 50 Hz und einer Impulsdauer von 100 Mikrosekunden, vorzugsweise zur Stimulation von Muskelpartien, insbesondere nach einem Schlaganfall;
3) Rechteckimpulse mit einer Frequenz von 100 Hz und einer Impulsdauer von 100 Mikrosekunden, vorzugsweise zur transcutanen Elektronervenstimulation, insbesondere zur Stimulation des Musculus temporalis oder bei Schmerzen wie Kopfschmerzen oder Migräne;
4) Rechteckimpulse mit einer Frequenz von 2 Hz und einer Impulsdauer von 100 Millisekunden, vorzugsweise zur funktionellen Elektromyostimulation des Muskulus corpus cavernosum;
5) Rechteckimpulse mit einer Frequenz von 2 Hz und einer Impulsdauer von 30 Millisekunden, vorzugsweise zur funktionellen Elektromyostimulation des Muskulus corpus cavernosum.

Fig. 3 stellt ein Funktionsschema zur Festlegung eines vorbestimmten Frequenzmusters dar.

In Schritt S1 gibt der Benutzer über die Stellung des Schalters 2 vor, ob er eine Dauerbetrieb wünscht (Stellung 3 des Rastschalters) oder eine zeitlich begrenzte Betriebsdauer von beispielsweise 15 Minuten (Stellung 1) oder 30 Minuten (Stellung 2).

In Schritt S2 wird abgefragt, in welcher Stellung sich der Wechselschalter 5 (nicht dargestellt) am Triggereingang 13 befindet. In Stellung 5.1. liegt ein Dauer- oder Intervallbetrieb vor, während in Stellung 5.2. ein Triggerbetrieb gewählt wird.

In Schritt S3 wird je nach Stellung des Schalters 3 entweder ein Dauerbetrieb gewählt (Stellung 1) oder ein Intervallbetrieb (Stellung 2). Für den Triggerbetrieb ist Schalter 3 ohne Bedeutung.

Bei einer bevorzugten Ausführungsform bedeutet ein Intervallbetrieb, daß das Gerät während 6 Sekunden das Frequenzmuster ausgibt, gefolgt von einem Pauseninterval von 5 Sekunden. Über die Geräteelektronik können jedoch auch andere Zeiten vorgegeben bzw. verstellt werden. Im Dauerbetrieb wird das gewählte Frequenzmuster ständig ausgegeben. Im Triggerbetrieb wird gemäß dem gewählten Betriebszustand entweder ein einziger Stromimpuls oder ein vorbestimmte Anzahl von Stromimpulsen ausgegeben.

Bei einer bevorzugten Ausführungsform sind in die Geräteelektronik einige bevorzugte Frequenzmuster wie beispielsweise die zuvor beschriebenen Frequenzmuster 1) bis 5) fest einprogrammiert. Hierzu dient bevorzugt eine anwenderspezifische Schaltung oder ein ASIC. Der Betriebszustand wird bevorzugt über Verstellung von geräteinternen Jumpern verstellt. Dies geschieht insbesondere aus Sicherheitsgründen, da das Gerät vom Benutzer nur zu einem fest vorgebbaren Benutzungszweck erworben werden soll und eine unbeabsichtigte Verstellung der Betriebszustände wirksam vermieden werden soll. Es kann jedoch auch vorgesehen sein, daß diese Jumper durch Gehäuseschalter ersetzt werden, so daß ein Benutzer den Betriebszustand ohne Öffnen des Gerätes vorgeben oder ändern kann.

Bevorzugt umfaßt die Geräteelektronik einen Jumper J1, der bei den Schritten S4 und S5 den Betriebszustand gemäß Fig. 4 gemeinsam mit den Geräteschaltern weiter festlegt. Befindet sich J1 in einer ersten Stellung, so ist das Gerät für die funktionelle Elektromyostimulation des Muskulus corpus cavernosum (FEMCC) geeignet ausgelegt, wobei die Impulsdauer von der Stellung des Schalters 4 abhängt (Betriebszustände 4 bzw. 5). Befindet sich J1 jedoch in einer zweiten Stellung, so ist das Gerät zur transcutanen Elektronervenstimulation ausgelegt (Betriebszustände 1 bzw. 3). Befindet sich schließlich J1 in einer dritten Stellung, so ist das Gerät geeignet zur Stimulation von Muskelpartien ausgelegt (Betriebszustand 2).

Eine bevorzugte, nichttherapeutische Anwendung des zuvor beschriebenen Gerätes betrifft ein Verfahren zur Verbesserung der körperlichen Erscheinung einer erwachsenen, männlichen Person.

Hierzu werden um die Peniswurzel, in einem Abstand von 2 - 3 cm, vorzugsweise zwei Streifenkontaktelektroden mit einer Größe von etwa 1 cm × 3 cm angebracht bzw. angeklebt. Anschließend wird an die Kontaktelektroden gemäß dem gewählten Betriebszustand eine Folge von Stromimpulsen angelegt, die selektiv den Muskulus corpus cavernosum stimulieren. Bevorzugt wird die Amplitude der Stromimpulse mit Hilfe des Drehpotentiometers 10 so gewählt, daß ein angenehmes Kribbeln auf der Haut verspürt wird.

Der Muskulus corpus cavernosum ist bei der Erektion des männlichen Gliedes maßgeblich beteiligt, da er die Erektion einleitet und unterhält, indem er selektiv für die Füllung des Schwellkörpers sorgt. Die Stimulation kann im Bedarfsfall oder dann vorgenommen werden, wenn dies von einem Trainingsplan vorgesehen ist. Durch die Stärkung des Muskels wird eine natürliche, spontane Erektion möglich, wodurch die körperliche Erscheinung der männlichen Person im Bedarfsfall verbessert wird, weil die Person männlicher wirken kann. Die besonders stimulierende bzw. aufbauende Wirkung von Stromimpulsen gemäß den vorstehenden Frequenzmustern 4) bzw. 5) war dem Stand der Technik weder entnehmbar noch durch diesen nahegelegt.

Bei anderen bevorzugten Verwendungsformen des Geräts werden die Kontaktelektroden im Bereich des Musculus temporalis der Person angebracht (Abbau von Spannungen und Kopfschmerzen bzw. Migräne), wodurch die körperliche Erscheinung dieser Person wegen der entspannenden Wirkung der Stromimpulse verbessert wird. Andere bevorzugte Verwendungs- bzw. Trainingszwecke betreffen die funktionelle Stimulation des Blasen- oder Mastdarmschließmuskels, also von isometrisch kontrahierenden Muskeln. Durch regelmäßige Stimulation bzw. Training wird die Wirksamkeit dieser Muskeln erhöht, wodurch sich ebenfalls die körperliche Erscheinung verbessern läßt.

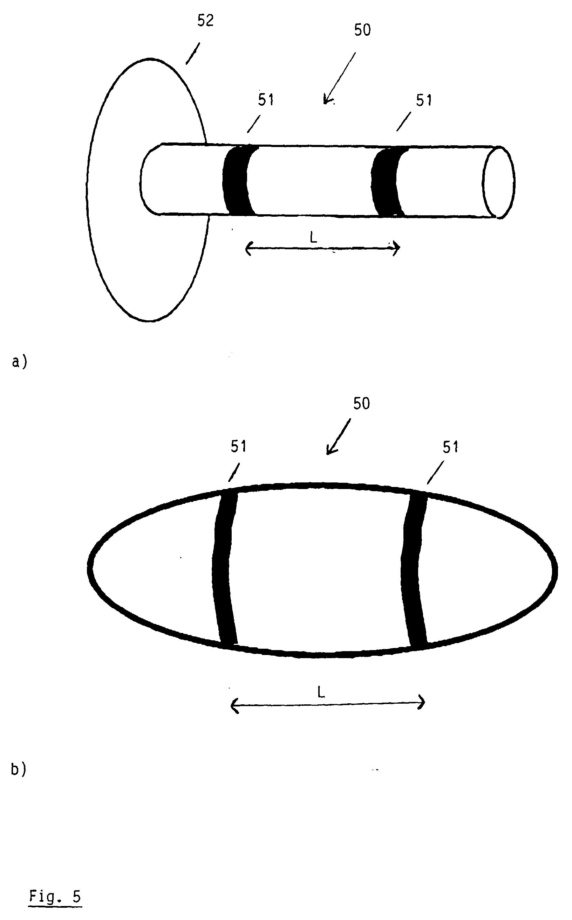

Figur 5 zeigt schließlich zwei bevorzugte Ausführungsformen von Elektrodenkörpern, d.h. Körpern, die oberflächlich mit Elektroden versehen sind und zur Einführung in Körperöffnungen bestimmt sind, beispielsweise in den Vaginal- oder Darmbereich. Die Elektroden dienen dabei zum Training bzw. zur Stimulierung von Muskel- oder Nervengeweben in solchen Körperöffnungen, vorzugsweise von Schließmuskelpartien. Bevorzugt werden hierzu zylinderförmige (siehe Figur 5a) oder ellipsenförmige (siehe Figur 5b) Elektrodenkörper. Die Gesamtlänge des Elektrodenkörpers 50 ist zur einfachen Handhabung geeignet ausgelegt, vorzugsweise 10 bis 20 cm.

Zweckmäßig ist der Elektrodenkörper 50 aus einem flexiblen Gummimaterial hergestellt. Vorzugsweise ist der Elektrodenkörper 50 jedoch aufpumpbar und aus einem widerstandsfähigen Material, so daß durch Ändern des Pumpdruckes die Form des Elektrodenkörpers individuell an die Person angepaßt werden kann. Zur Längeneinstellung können Balgabschnitte, vorzugsweise im Mittelbereich, vorgesehen sein.

Die Kontaktelektroden 51 verlaufen in Umfangsrichtung und sind vorzugsweise an einer Stelle unterbrochen, so daß bei einer Änderung des Pumpdruckes bzw. des Umfanges des Elektrodenkörpers 50 die Elektrode selbst nicht zerreißt. Hierzu ist die Elektrode 51 vorzugsweise dehnbar ausgelegt, beispielsweise durch einen Zick-Zackverlauf der Elektrodenbahn in Umfangsrichtung. Der Axialabstand der beiden Elektroden 51 ist an die Ausdehnung des zu trainierenden Muskel- bzw. Nervengewebes angepaßt und beträgt vorzugsweise 1 bis 2 cm. Der Endabstand der beiden Elektroden 51 wird dabei geeignet gewählt. Zur Verstellung des Elektrodenabstands kann der Balgabschnitt (nicht gezeigt) zwischen den beiden Elektroden 51 vorgesehen sein.

Damit der eingeführte Elektrodenkörper 50 nicht in der Körperöffnung verschwindet, wird bevorzugt frühzeitig ein Anschlag 52 auf den Elektrodenkörper aufgesetzt. Dieser Anschlag kann mit dem Elektrodenkörper verschraubt sein, bei einer bevorzugten Ausführungsform ist der Anschlag 52 jedoch als Ring aus Gummi mit einer großen Reibungskonstante ausgelegt, so daß der ringförmige Anschlag 52 seiner Axialverschiebung entgegenwirkt.

## Patentansprüche

1. Elektromedizinische Vorrichtung, mit den folgenden Merkmalen:
- einen Oszillator zur Erzeugung eines niederfrequenten Strom- bzw. Spannungsimpuls-Frequenzmusters,
- zumindest eine, bevorzugt zwei Kontaktelektroden (1), die mit einem Ausgang des Oszillators verbunden sind, insbesondere zur funktionellen Stimulation von Muskel- und/oder Nervengewebe.

2. Vorrichtung nach Anspruch 1, bei der das Austastverhältnis des Frequenzmusters (S6) kleiner als oder etwa gleich 0,25 ist.

3. Vorrichtung nach einem der vorhergehenden Ansprüche, mit einer Einrichtung (7, 8, 9) zum Verstellen des vorbestimmten Frequenzmusters in ein anderes vorbestimmtes Frequenzmuster.

4. Vorrichtung nach einem der vorhergehenden Ansprüche, mit
- zumindest einer Meßelektrode zum Abgreifen eines Signals von einer Körperstelle einer Person,
- einem Triggereingang (13), mit dem die Meßelektrode verbunden ist und an dem das Signal anliegt, und
- einer Verstärkerschaltung (10) zur Verstärkung des abgegriffenen Signals.

5. Vorrichtung nach Anspruch 4, bei der der Oszillator nur dann Strom- bzw. Spannungsimpulse ausgibt, wenn das verstärkte Signal einen vorbestimmten Schwellenwert übersteigt.

6. Vorrichtung nach einem der vorhergehenden Ansprüche, bei der der Oszillator bevorzugt trapezförmige Stromimpulse ausgibt, deren Anstiegszeit etwa 25 % der Gesamtpulsdauer beträgt, wobei bevorzugt eine netzunabhängige Spannungsversorgung zur Leistungsversorgung vorgesehen ist.

7. Verwendung einer elektromedizinischen Vorrichtung nach einem der vorhergehenden Ansprüche zur gezielten Stimulation bzw. zum Training von Muskelund/oder Nervengewebe.

8. Verwendung einer elektromedizinischen Vorrichtung nach einem der Ansprüche 1 bis 6 zur gezielten Stimulation bzw. Training des Musculus Corpus Cavernosum bei einer erwachsenen, männlichen Person, bzw. zur Verbesserung der körperlichen Erscheinung einer erwachsenen, männlichen Person, wobei die zumindest eine Kontäktelektrode (1) in der Nähe der Peniswurzel angebracht wird, so daß der Musculus Corpus Cavernosum funktionell stimuliert wird, wodurch die körperliche Erscheinung der Person verbessert wird.

9. Verwendung einer elektromedizinischen Vorrichtung nach einem der Ansprüche 1 bis 6 zur Verbesserung der körperlichen Erscheinung einer Person, wobei die zumindest eine Kontaktelektrode (1) am Musculus temporalis der Person angebracht wird, zur funktionellen Stimulation des Musculus temporalis.

10. Verfahren zur Verbesserung der körperlichen Erscheinung einer erwachsenen, männlichen Person, bei dem
- zumindest eine Kontaktelektrode (1) nahe der Peniswurzel der Person angebracht wird, und
- ein Stromimpuls-Frequenzmuster (S6) an die zumindest eine Kontaktelektrode (1) angelegt wird, so daß der Musculus Corpus Cavernosum funktionell stimuliert wird, wodurch die körperliche Erscheinung der Person durch verbesserte Füllung des Schwellkörpers verbessert wird, wobei
- bevorzugt zwei Kontaktelektroden (1) in etwa symmetrisch bezüglich der Peniswurzel und in einem Abstand von etwa 2 bis 3 cm von dieser entfernt angeordnet werden.
